(19) Europäisches Patentamt
European Patent Office
Office européen des brevets

(11) EP 0 758 006 B1

(12) **FASCICULE DE BREVET EUROPEEN**

(45) Date de publication et mention de la délivrance du brevet:
**26.02.2003 Bulletin 2003/09**

(51) Int Cl.[7]: **C09C 3/04**, A61K 7/00

(21) Numéro de dépôt: **96401744.6**

(22) Date de dépôt: **07.08.1996**

(54) **Procédé de passivation d'un pigment minéral photoréactif par traitement par un plasma froid d'un gaz organique et composition cosmétique ou pharmaceutique, stable à la lumière, contenant au moins un pigment ainsi passivé**

Verfahren zur Passivierung eines photoreaktiven Mineralpigments durch Behandlung mittels eines kalten organischen Plasmas, und lichtechte, kosmetische oder pharmazeutische Zusammensetzung enthaltend mindenstens ein auf diese Weise passiviertes Pigment

Process for passivating a photoreactive mineral pigment by an organic cold plasma treatment and lightfast cosmetic or pharmaceutical composition containing at least one thus passivated pigment

(84) Etats contractants désignés:
**DE ES FR GB IT**

(30) Priorité: **09.08.1995 FR 9509675**

(43) Date de publication de la demande:
**12.02.1997 Bulletin 1997/07**

(73) Titulaire: **L'OREAL, S.A.**
**75008 Paris (FR)**

(72) Inventeurs:
- **Leveque, Jean-Luc**
  **93340 Le Raincy (FR)**
- **Leroy, Frédéric**
  **92210 Saint Cloud (FR)**
- **Mellul, Myriam**
  **94240 L'Hay Les Roses (FR)**
- **Duvault, Yolanda**
  **93600 Aulnay-sous-Bois (FR)**

(74) Mandataire: **Tanty, François et al**
**Nony & Associés,**
**3, Rue de Penthièvre**
**75008 Paris (FR)**

(56) Documents cités:
- **POLYMERIC MATERIALS: SCIENCE AND ENGINEERING, vol. 62, 1990, AMER. CHEM. SOC., pages 452-456, XP000568436 T. KOBAYASHI ET AL.: "PLASMA TREATMENT OF TIO2 AND QUINACRIDONE RED PIGMENTS AND THEIR DISPERSION BEHAVIOUR IN WATER BORNE PAINT"**
- **JOURNAL OF COATINGS TECHNOLOGY, vol. 64, no. 809, Juin 1992, BLUE BELL, PA, USA, pages 41-46, XP000568427 T. KOBAYASHI ET AL.: "EFFECT OF PLASMA TREATMENT OF TITANIUM DIOXIDE AND QUINACRIDONE RED PIGMENTS ON DISPERSION BEHAVIOUR IN WATER-SOLUBLE ACRYLIC RESINS"**
- **DATABASE WPI Week 8446 Derwent Publications Ltd., London, GB; AN 84-285013 XP002000576 & JP-A-59 175 409 (SHISEIDO) , 4 Octobre 1984 & JP-A-84 175 409 (...)**
- **CHEMICAL ABSTRACTS, vol. 86, no. 16, 18 Avril 1977 Columbus, Ohio, US; page 77; colonne R; XP002000575 & KHIM. IND. (SOFIA), vol. 48, no. 10, 1976, pages 441-444, VIDENOV ET AL.: "PREPARATION OF IRON OXIDE PIGMENTS UNDER LOW-TEMPERATURE PLASMA CONDITIONS"**
- **DATABASE WPI Week 8148 Derwent Publications Ltd., London, GB; AN 81-88474D XP002000577 & JP-A-56 134 784 (CANON) , 21 Octobre 1981**

EP 0 758 006 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- DATABASE WPI Week 9424 Derwent Publications Ltd., London, GB; AN 94-196344 XP002000578 & JP-A-06 134 296 (EC KAGAKU & ITOCHU FINE CHEM.) , 17 Mai 1994
- DATABASE WPI Week 8351 Derwent Publications Ltd., London, GB; AN 83-848785 XP002000579 & SU-A-242 775 (BRIT. TITAN PROD.) , 24 Septembre 1969
- DATABASE WPI Week 8846 Derwent Publications Ltd., London, GB; AN 88-325893 XP002000580 & JP-A-63 239 102 (NIPPON STEEL) , 5 Octobre 1988
- DATABASE WPI Week 8640 Derwent Publications Ltd., London, GB; AN 86-262052 XP002000581 & JP-A-61 190 567 (SHISEIDO) , 25 Août 1986

## Description

La présente invention a pour objet un procédé de passivation d'un pigment minéral photoréactif ainsi qu'une composition cosmétique ou pharmaceutique, stable à la lumière, contenant au moins un pigment minéral sous forme passivée.

Selon la présente demande, on entend par "stable à la lumière", pour un pigment donné ou une composition le contenant, l'absence de variation de couleur ainsi que l'absence de formation d'espèces oxydoréductrices après une exposition prolongée à la lumière.

Par ailleurs, on entend par "forme passivée" une forme stable à la lumière d'un pigment minéral, obtenue à partir d'une forme photoréactive de ce pigment minéral.

On entend enfin selon la présente demande par "traitement de passivation" un traitement permettant d'obtenir, pour un pigment minéral photoréactif donné, la forme passivée telle que définie ci-dessus.

L'utilisation d'un pigment minéral photoréactif dans une composition cosmétique ou pharmaceutique soulève de sérieux problèmes de stabilité à la lumière d'un tel pigment et, par voie de conséquence, de la composition le contenant. Par exemple, parmi les pigments minéraux photoréactifs les plus connus et utilisés on peut citer le dioxyde de titane qui par exposition à la lumière, en particulier aux rayons ultra-violets, subit une réaction que l'on peut schématiser ainsi :

$$TiO_2 \xrightarrow{h\upsilon} TiO^{\square}_2 + 1e^-$$

$$Ti^{4+}_{\text{incolore}} + 1\ e^- \longrightarrow Ti^{3+}_{\text{gris noir}}$$

($^{\square}$ symbolisant une lacune électronique)

Cette réaction provoque donc une variation de couleur du pigment $TiO_2$ et de la composition le contenant, celle-ci pouvant d'ailleurs se poursuivre au sein de cette dernière, conduisant à une dégradation potentielle d'autres composants tels que des huiles végétales, etc.

D'une manière générale, la présence d'un pigment minéral photoréactif dans une composition cosmétique ou pharmaceutique est préjudiciable car elle conduit à un changement de couleur de la composition et/ou à la formation d'espèces oxydoréductrices dans cette dernière, nécessitant l'emploi de divers moyens de protection tels qu'un conditionnement étanche à la lumière ou l'enrobage des particules de pigment, par exemple à l'aide d'une association alumine/silice. Or, l'emploi d'un conditionnement étanche à la lumière va à l'encontre de l'attente du consommateur désireux de pouvoir apprécier l'apparence d'une composition cosmétique ou pharmaceutique lors de son achat. Par ailleurs, l'enrobage des particules du pigment a pour inconvénient de modifier l'indice de réfraction et donc les propriétés pigmentaires souhaitées.

Si ces dernières années, les techniques plasma ont connu un essor important, celles-ci ont été essentiellement dirigées dans l'optique d'obtenir, sur la surface de divers matériaux, le dépôt d'un film polymérique. Il est ainsi connu de traiter la surface d'un pigment organique ou minéral par un plasma en vue d'en améliorer la dispersibilité notamment dans une composition cosmétique. Cette technique a été appliquée notamment au noir de carbone qui est très utilisé dans les eye-liners mais est difficile à disperser du fait de sa faible granulométrie.

Dans ce contexte, on peut citer la demande JP 57 159,856 qui décrit l'utilisation dans divers domaines de particules de noir de carbone dont la dispersibilité dans l'eau a été améliorée par traitement par un plasma froid d'oxygène gazeux. On peut également citer la demande JP 63 165.461 qui décrit la formation d'un film de silicone à la surface de particules d'une poudre à l'aide d'un plasma froid, le film de silicone conférant un caractère hydrophobe à la surface des particules ainsi revêtues. Enfin, on peut citer la demande JP 84 175.409 qui décrit une composition cosmétique comprenant au moins un pigment organique ou minéral par exemple du dioxyde de titane ou du noir de carbone, ayant été traité par un plasma à l'aide d'un appareil de la Société SANKYO DENGYO K.K., commercialisé sous la dénomination de Practive Line®. Par ce type de traitement, les pigments organiques et minéraux présentent une meilleure dispersibilité en solution aqueuse et n'ont plus tendance à former des agrégats. Du fait de cette meilleure dispersibilité, les compositions cosmétiques contenant de tels pigments ont donc un aspect plus homogène et par conséquent une teinte moins foncée à l'application.

On a maintenant trouvé de manière surprenante et inattendue que lorsque l'on traitait une classe particulière de pigments, à savoir des pigments minéraux photoréactifs par un plasma froid d'un gaz organique, les pigments résultants, ou pigments passivés, ne présentaient plus de propriétés pro-oxydantes ou, du moins, celles-ci étaient très fortement atténuées.

Grâce à ce traitement des pigments minéraux photoréactifs, il est donc maintenant possible d'obtenir des

compositions cosmétiques ou pharmaceutiques ne présentant plus d'effet notable de coloration dans le temps.

La présente invention a donc pour objet, un procédé de passivation d'un pigment minéral photoréactif par un plasma froid d'un gaz organique constitué d'un hydrocarbure perfluoré ce procédé étant tel que défini dans les revendications annexées.

Parmi les pigments minéraux photoréactifs susceptibles d'être passivés par le procédé selon l'invention, on peut citer $TiO_2$, ZnO, $Ce_2O_3$, CdS, CaAs, $ZrO_2$, $Fe_2O$, FeO et $Fe_2O_3$. Ces pigments ont une taille de l'ordre du micromètre ou du nanomètre, et une morphologie du type lamellaire aciculaire ou sphérique.

Comme hydrocarbure perfluoré en $C_1$-$C_4$, on utilise par exemple le tétrafluorométhane.

Selon une forme de réalisation préférée du procédé on utilise un mélange d'un hydrocarbure perfluoré en $C_1$-$C_4$ et d'un gaz inerte tel que l'argon.

Le procédé selon l'invention est mis en oeuvre au sein d'une enceinte à vide dans laquelle sont placés les échantillons de pigments minéraux photoréactifs à traiter, celle-ci étant reliée à une arrivée du gaz organique, à un générateur de micro-ondes et à une pompe à vide.

Les caractéristiques du rayonnement micro-ondes sont de préférence comprises entre 300 et 30000 MHz et la puissance restituée entre 1 et 5000 W.

La pression du gaz organique est de préférence comprise entre 0,1 bar et 0,01 millibar (soit entre $0{,}1.10^5$ et 1 Pa) et son débit entre 0,1 et $1.10^{-2}$ l/min.

La durée du traitement est généralement comprise entre 1 et 60 minutes.

Les pigments minéraux après traitement de passivation ne présentent pas sensiblement d'augmentation du diamètre des particules, celui-ci étant compris entre environ 20 nm et 5 $\mu$m suivant l'espèce minérale considérée.

Il sera donné ci-après un exemple de traitement d'un pigment minéral photoréactif par un plasma froid de tétrafluorométhane.

La présente invention a également pour objet une composition cosmétique ou pharmaceutique, stable à la lumière, telle que définie dans les revendications annexées.

Elle vise également l'utilisation d'un pigment minéral, stable à la lumière se présentant sous forme passivée telle que définie dans les revendications annexées, dans un produit cosmétique ou pharmaceutique.

Comme ceci a été préalablement précisé, les compositions selon l'invention contenant de tels pigments minéraux sous forme passivée ne présentent plus de variation de coloration ou du moins une variation de couleur très fortement diminuée.

Par ailleurs, la perte des propriétés pro-oxydantes des pigments minéraux photoréactifs présente également un grand intérêt dans la mesure où les lipides présents dans les compositions sont protégés de l'oxydation de même que les lipides et les protéines de la peau sur laquelle sont appliquées les compositions.

Les compositions cosmétiques selon l'invention sont essentiellement celles concernant le maquillage du visage, c'est-à-dire les fards à paupières, eye-liners, mascaras, poudres, fonds de teint, fards à joues, "blush", crèmes teintées, rouges à lèvres, sticks à lèvres ou sticks anti-cernes, mais également le maquillage des cheveux et le maquillage des ongles notamment les vernis à ongles anhydres et aqueux. Les compositions selon l'invention, selon la nature du ou des pigments, peuvent être également non colorés et se présenter sous forme de produit solaires, de crèmes et autres produits cosmétiques de protection quotidienne.

Dans les compositions selon l'invention, la proportion en pigment minéral sous forme passivée est comprise de préférence entre environ 0,01 et environ 40 % en poids par rapport au poids total de la composition, et plus particulièrement entre environ 0,1 et 25 %.

Les compositions cosmétiques ou pharmaceutiques selon l'invention peuvent en outre contenir des pigments additionnels minéraux non photoréactifs ou des pigments organiques ainsi que des charges usuels en cosmétique.

Parmi les pigments minéraux non photoréactifs, on peut citer, à titre d'exemple :

- le noir de carbone,
- le violet de manganèse,
- les oxydes de chrome,
- le carbonate de calcium,
- les pigments bleus ferrique - ferrocyanate.

Parmi les pigments organiques, on peut citer, en particulier, les suivants :

- D & C red n° 19 (CI 45170);
- D & C red n° 9 (CI 15585);
- D & C red n° 21 (CI 45380);
- D & C orange n° 4 (CI 15510);
- D & C orange n° 5 (CI 45370);

- D & C red n° 27 (CI 45410);
- D & C red n° 13 (CI 15630);
- D & C red n° 7 (CI 15850-1);
- D & C red n° 6 (CI 15850-2);
- D & C yellow n° 5 (CI 19140);
- D & C red n° 36 (CI 12085);
- D & C orange n° 10 (CI 45425) ;
- D & C yellow n° 6 (CI 15985);
- D & C red n° 30 (CI 73360);
- D & C red n° 3 (CI 45430);
- le noir de carbone (CI 77266); et
- les laques à base de carmin de cochenille (CI 75470).

Les pigments additionnels tels que définis ci-dessus peuvent être présents dans la composition selon l'invention en une proportion de 0,01 à 25 % en poids par rapport au poids total de la composition, et de préférence entre 0,1 et 15 % en poids.

Les charges sont choisies notamment parmi :

- la silice,
- le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoelithe, lépidolithe, biotite) ou d'origine synthétique. Les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de forme isotrope ayant des dimensions généralement inférieures à 30 µm, et qui possède de bonnes propriétés d'absorption des corps gras ;
- le nitrure de bore ;
- le carbonate de calcium précipité qui, sous forme de particules de dimensions inférieures à 10 µm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, etc. Ces savons, présents généralement sous la forme de particules ayant des dimensions inférieures à 10 µm ont un toucher onctueux et facilitent l'adhérence de la poudre sur la peau ;
- les poudres de polymères synthétiques, tels que le polyéthylène, les polyesters (par exemple isophtalate ou téréphtalate de polyéthylène), les polyamides, sous la forme de particules ayant des dimensions inférieures à 50 µm, qui possèdent des propriétés absorbantes, et permettent de conférer à la peau un aspect velouté, les poudres de méthacrylate ;
- des microsphères creuses, telles que par exemple les microsphères creuses Expancel® vendues par la Société KEMANORD PLAST AB et décrites dans le brevet français 86 09289 (2.600.532).

Les charges peuvent être représentées en une proportion de 0,01 à 90 % en poids par rapport au poids total de la composition.

Les compositions selon la présente invention peuvent notamment se présenter sous forme d'émulsion huile-dans-l'eau ou eau-dans-l'huile, ou sous forme d'une suspension en milieu solvant, ou encore sous forme de poudre libre, de poudre compactée, ou d'un solide ou d'une pâte anhydre. Les modes opératoires pour la préparation de ces différents types de composition sont bien connus de l'homme de l'art.

Lorsqu'elles sont utilisées sous forme d'émulsion, les compositions selon l'invention peuvent contenir des agents tensio-actifs bien connus de l'état de la technique. Ces tensio-actifs peuvent être présents en une proportion de 0,01 à 30 % en poids par rapport au poids total de la composition.

Une réalisation particulièrement préférée consiste à préparer des émulsions anioniques ou non-ioniques en utilisant des agents tensio-actifs anioniques ou non-ioniques dans des proportions de préférence comprises entre 2

et 30 % en poids par rapport au poids total de la composition.

Parmi les agents tensio-actifs anioniques qui peuvent être utilisés seuls ou en mélange, on peut citer en particulier les sels alcalins, les sels d'ammonium, les sels d'amines ou les sels d'aminoalcools des composés suivants :

- les alcoylsulfates, alcoyléther sulfates, alcoylamides sulfates et éthers sulfates, alcoylarylpolyéthersulfates, monoglycérides sulfates,
- les alcoylsulfonates, alcoylamides sulfonates, alcoylarylsulfonates, α-oléfines sulfonates, paraffines sulfonates,
- les alcoylsulfosuccinates, alcoyléthersulfosuccinates, alcoylamides sulfosuccinates,
- les alcoylsulfosuccinamates,
- les alcoylsulfoacétates, les alcoylpolyglycérol carboxylates,
- les alcoylphosphates/alcoylétherphosphates,
- les acylsarcosinates, alcoylpolypeptidates, alcoylamidopolypeptidates, acyliséthionates, alcoyllaurates.

Le radical alcoyle ou acyle dans tous ces composés désigne généralement une chaîne de 12 à 18 atomes de carbone.

D'autres agents tensio-actifs anioniques sont constitués par les sels d'acides gras tels que les acides oléique, ricinoléique, palmitique, stéarique, les acides d'huile de coprah ou d'huile de coprah hydrogénée et notamment les sels d'amines tels que les stéarates d'amines.

On peut également citer :

- les acyl lactylates dont le radical acyle comprend de 8 à 20 atomes de carbone,
- les acides carboxyliques d'éthers polyglycoliques répondant à la formule :

$$Alk\text{-}(OCH_2\text{-}CH_2)_n\text{-}OCH_2\text{-}COOH$$

sous forme acide ou salifiée où le substituant Alk correspond à une chaîne linéaire ayant de 12 à 18 atomes de carbone et où n est un nombre entier compris entre 5 et 15.

Parmi les tensio-actifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone. On peut également citer des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose.

D'autres composés entrant dans cette classe sont les produits de condensation d'un α-diol, d'un monoalcool, d'un alcoylphénol, d'un amide ou d'un diglycolamide avec le glycidol ou un précurseur de glycidol.

Les tensio-actifs non-ioniques principalement utilisés sont des alcools polyéthoxylés ou polyglycérolés tels que les alcools stéarylique, cétylstéarylique ou oléique polyéthoxylés.

Les tensio-actifs anioniques préférentiellement utilisés sont des stéarates d'amines.

Les compositions selon l'invention peuvent également se présenter sous forme d'un gel, d'une solution aqueuse ou hydroalcoolique d'un ou plusieurs polymères hydrosolubles tels que les dérivés de l'acide polyacrylique ou sous forme de gels émulsionnés obtenus par dispersion d'huiles dans des gels à l'aide d'émulsionnants tels que les Pemulens® de la Société GOODRICH.

Les compositions selon la présente invention peuvent en outre contenir des ingrédients usuels choisis parmi des adoucissants, des conservateurs, des séquestrants, des parfums, des épaississants, des agents de cohésion, des polymères ainsi que des agents alcalinisants ou acidifiants, des hydratants et des actifs hydrosolubles.

Les épaississants utilisables peuvent être naturels ou synthétiques. Parmi les épaississants naturels, on peut citer les gommes de diverses sortes telles que les gommes arabique, de guar ou de caroube. Parmi les épaississants de synthèse, on peut citer les dérivés cellulosiques comme l'hydroxyéthylcellulose, la carboxyméthylcellulose, les dérivés de l'amidon, les dérivés d'éthers de cellulose possédant des groupes ammonium quaternaires, les polysaccharides cationiques, les sels de polymères acryliques ou méthacryliques, les polyènes ou les polysiloxanes.

On peut également obtenir un épaississement des compositions par mélange de polyéthylèneglycol et de stéarate et/ou de distéarate de polyéthylèneglycol ou par un mélange d'esters phosphoriques et d'amides gras.

La phase huileuse peut représenter de 0,1 à 50 % en poids par rapport au poids total de l'émulsion.

Elle peut être constituée d'huiles, et/ou de cires.

Les cires et les huiles peuvent être d'origine végétale, animale, minérale ou synthétique.

Parmi les huiles végétales, on peut citer l'huile de jojoba, l'huile d'olive, l'huile d'amande douce, l'huile d'avocat, l'huile de coco, l'huile de germe de blé, l'huile de maïs, l'huile de palme, l'huile de sésame, l'huile de soja, l'huile d'argan, l'huile d'onagre, l'huile de bourrache et les huiles essentielles.

Parmi les huiles animales on peut notamment citer l'huile de poisson.

Parmi les huiles minérales on peut citer l'huile de vaseline et d'isohexadécane.

Parmi les huiles synthétiques on peut mentionner les palmitates d'éthyle et d'isopropyle, le palmitate d'éthyl-2 hexyle, les myristates d'alkyle tels que le myristate d'isopropyle, de butyle, de cétyle, le stéarate d'hexyle, les triglycérides des acides octanoïque et décanoïque, le ricinoléate de cétyle et l'octanoate de stéaryle, les huiles de silicone, les huiles perfluorées, les huiles de silicone fluorées.

La phase huileuse peut par ailleurs contenir des colorants, des filtres solaires, des antioxydants, des conservateurs et des principes actifs lipophiles.

Les compositions anhydres selon l'invention, qui peuvent se présenter sous forme de poudre libre ou compactée, de fard solide, pâteux ou liquide, peuvent contenir un liant qui peut représenter de préférence de 0,01 à 95 % en poids par rapport au poids total de la composition.

Parmi les agents liants, on peut citer notamment les huiles animales, végétales ou synthétiques, les mélanges d'huile(s) et de cire(s) et en particulier l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, etc. ; des huiles d'hydrocarbures, telles que des huiles de paraffine, le squalane, la vaseline, etc. ; des esters tels que le myristate d'isopropyle, le palmitate d'isopropyle, le stéarate de butyle, le laurate d'hexyle, l'isononanate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine, etc. ; des huiles de silicone comme les poly-méthylsiloxanes, les poly-méthylphénylsiloxanes, des polysiloxanes modifiés par des acides gras, des polysiloxanes modifiés par des alcools gras, des polysiloxanes modifiés par des polyoxyalkylènes, des silicones fluorées, etc. ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, etc. ; des alcools gras supérieurs tels que le cétanol, l'alcool stéarylique, l'alcool oléique, etc. ; les cires peuvent être choisies notamment parmi la cire de carnauba, la cire de candelilla, la cire d'abeille, la cire de baleine, des lanolines, des cires microcristallines, etc.

Le liant peut contenir en outre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa).

Parmi les huiles volatiles pouvant être présentes comme agents d'étalement dans la composition de l'invention, on citera par exemple les huiles de silicone telles que l'hexaméthyldisiloxane, le cyclopentadiméthyl-siloxane, le cyclotétraméthylsiloxane, des huiles fluorées comme celle commercialisée sous la dénomination GALDEN® (MONTEFLUOS) ou des huiles isoparaffiniques telles que celles commercialisées sous la dénomination ISOPAR® (E, G, L ou H ; EXXON CHEMICAL).

Comme mentionné ci-dessus, les compositions selon l'invention peuvent également se présenter sous forme d'un vernis à ongles anhydre ou aqueux.

Lorsque les compositions se présentent sous forme d'un vernis à ongles anhydre, le système solvant représente environ de 55 % à 90 % en poids par rapport au poids total du vernis.

Le système solvant est généralement constitué par un mélange de divers solvants organiques volatils tels que l'acétone, l'acétate d'éthyle, l'acétate de butyle, l'acétate de méthoxy-2 éthyle, la méthyléthylcétone, la méthylisobutylcétone, l'acétate de méthyle, l'acétate d'amyle et l'acétate d'isopropyle.

Le système solvant peut également comprendre un diluant tel que l'hexane ou l'octane ou encore un hydrocarbure aromatique tel que le toluène ou le xylène en une proportion de 10 à 35 % en poids par rapport au poids total du vernis.

La matière filmogène du vernis est généralement présente à une concentration comprise entre 5 et 20 % en poids par rapport au poids total du vernis.

Parmi les matières filmogènes on peut notamment citer les nitrocelluloses du type "RS" ou "SS" et en particulier la nitrocellulose type 1/4"RS", la nitrocellulose type 1/2"RS", la nitrocellulose type 1/2"SS" et la nitrocellulose type 3/4"RS".

Les vernis contiennent également un agent plastifiant généralement présent à une concentration comprise entre 2 et 10 % en poids par rapport au poids total du vernis. Parmi ceux-ci on peut notamment mentionner le phosphate de tricrésyle, le benzoate de benzyle, le citrate de triéthyle, le citrate de tributyle, l'acétylcitrate de triéthyle, l'acétylcitrate de triétyl-2 hexyle, le phtalate de diamyle, le camphre.

Les vernis selon l'invention contiennent également une résine généralement présente à une concentration

comprise entre 0,5 et 15 % en poids par rapport au poids total du vernis.

Parmi les nombreuses résines utilisables on peut notamment citer les résines du type aryl-sulfonamide formaldéhyde ou aryl-sulfonamide époxy notamment les résines connues sous les dénominations commerciales SANTOLITE MHP® et SANTOLITE MS 80 %®.

Lorsque les vernis à ongles se présentent sous forme aqueuse, ils contiennent une dispersion d'une substance filmogène synthétique dans laquelle divers additifs usuels peuvent être ajoutés tels qu'une matière filmogène, un épaississant, un régulateur de pH, un réticulant, un anti-mousse, etc.

Comme dispersion aqueuse synthétique, on peut, entre autre, utiliser des dispersions d'acétate de polyvinyle, de polyuréthanne, de polymères ou copolymères acryliques et de copolymères d'acétate de polyvinyle.

La dispersion aqueuse synthétique représente environ de 10 à 80 % en poids du vernis.

La matière filmogène est généralement présente à une concentration comprise entre 5 et 20 % en poids par rapport au poids total du vernis.

Parmi les matières filmogènes on peut notamment citer les dérivés de cellulose solubles dans l'eau.

Les vernis selon l'invention peuvent également contenir une résine généralement présente à une concentration comprise entre 0,5 et 15 % en poids par rapport au poids total du vernis.

Parmi les résines utilisables on peut notamment citer les résines du type acrylique, styrénique, acrylate-styrénique et vinylique.

Les vernis à ongles anhydres ou aqueux selon l'invention peuvent également contenir des adjuvants couramment utilisés dans les vernis à ongles tels que par exemple des filtres U.V.

## METHODES DE DETERMINATION DE LA PHOTOSTABILITE

A - Les compositions selon la présente invention telles que décrites ci-dessus présentent de préférence, une photostabilité globale caractérisée par un photobleuissement $\Delta E = \sqrt{\Delta L^2+\Delta a^2+\Delta b^2}$, inférieur à 15 et de préférence inférieur à 10, mesuré à l'aide d'un chromamètre MINOLTA après 1 heure d'irradiation au moyen d'une source lumineuse émettant des rayons UV-A d'intensité 765 W/m$^2$.

Le test colorimétrique de mesure de la photostabilité est effectué selon le mode opératoire suivant. On place un échantillon dans une cuve colorimétrique puis on mesure au chromamètre MINOLTA, au temps $t_0$ (avant irradiation), la couleur de l'échantillon avec les paramètres L, a, et b. On irradie ensuite la composition à tester pendant 1 heure au moyen d'un dispositif du type CPS vendu par la Société HERAEUS muni d'une lampe de xénon d'intensité 760 W/m$^2$. L'échantillon est placé à une distance d'environ 27 cm de la lampe. On mesure la couleur de l'échantillon ainsi irradié et on calcule enfin, la variation de couleur de la composition après 1 heure d'irradiation ($\Delta E$). Dans le cas du dioxyde de titane, le $\Delta E$ traduit le passage de $Ti^{4+}$ à $Ti^{3+}$. Plus le $\Delta E$ est important, plus la composition est instable.

B - Les compositions selon l'invention présentent également, une photostabilité globale, caractérisée par un dégagement de pentane à une teneur, de préférence, inférieure à 20 µg, et plus particulièrement inférieur à 15 µg, après photo-oxydation en présence d'un réactif comportant des chaînes linoléiques (tel que la vitamine F) au moyen d'une source lumineuse émettant des rayons UV-A d'intensité 89 W/m$^2$ et des rayons UV-B d'intensité 0,05 W/m$^2$, pendant une durée maximale de 4 heures.

Le test de mesure de la photostabilité par dégagement de pentane est effectué selon le mode opératoire suivant. On utilise un appareil chromatographique du type CHAINE HEAD SPACE commercialisé par la Société PERKIN-ELMER, muni d'un tube de 20 ml étanche dans lequel est introduit la composition à tester puis serti par une capsule. Il sert à mesurer la teneur en pentane. On utilise comme dispositif d'irradiation un appareil du type SUNTEST, CPS modèle B150 commercialisé par la Société HERAEUS, muni d'un filtre en verre de diamètre 3,7 cm. On applique sur le filtre, de façon homogène, 0,1 + 0,005 g de composition à tester. On place le filtre dans le tube que l'on sertit ensuite. On irradie la composition pendant une durée maximale de 4 heures, puis on retire le tube et on mesure au moyen de l'appareil la teneur en pentane formé par photo-oxydation.

Les exemples suivants permettent d'illustrer l'invention, à titre non limitatif.

### *Exemple 1 : Traitement du $TiO_2$ par plasma post-décharge froid $CF_4$*

Dans une enceinte à vide munie d'une entrée et d'une sortie de fluide on place, en regard de l'entrée de fluide de l'enceinte, 10 g de $TiO_2$ ayant un diamètre moyen de particules inférieur ou égal à 200 nm. On établit alors le vide à l'intérieur de l'enceinte à l'aide d'une pompe à vide à palettes placée à la sortie de fluide de l'enceinte à vide et on maintient en marche la pompe pendant la durée du traitement. Le vide obtenu est de 0,1 millibar (soit 10 Pa).

En regard de l'entrée de fluide, à l'extérieur de l'enceinte à vide, on place une cavité micro-ondes reliée à un générateur. On fait circuler dans cette cavité, puis dans l'enceinte à vide, un flux d'un mélange gazeux de $CF_4$ à 2 % dans l'argon ayant une pression de 0,1 millibar (soit 10 Pa) et un débit de 0,1 l/mn. Un plasma est obtenu dans cette cavité par irradiation, à température ambiante, de ce mélange gazeux par rayonnement micro-ondes de fréquence

2450 MHz et de puissance restituée égale à 400 W.

Le $TiO_2$ est ainsi exposé au flux de plasma pendant un temps de 2 mn. On ramène ensuite la pression dans l'enceinte à la pression ambiante pour récupérer le $TiO_2$ passivé.

Selon le même mode opératoire que ci-dessus, on a procédé au traitement de passivation des pigments minéraux suivants : ZnO, $ZrO_2$, $Fe_2O$, FeO et $Fe_2O_3$.

Exemple A

On prépare une crème ayant la composition suivante :

- Alcool cétéarylique et Ceteareth-33 5,00 %
- Stéarate de glycéryle 1,00 %
- Alcool cétylique 1,00 %
- Huile de jojoba 6,00 %
- Acide linoléique et acide linolénique (50/50) 6,00 %
- $TiO_2$ passivé selon l'exemple 1 2,50 %
- Conservateurs qs
- Eau qsp 100,00 %

Cette composition présente une photostabilité globale caractérisée par :

- un $\Delta E$ égal à 10,
- un taux de pentane d'environ 15.

Si dans la composition de l'exemple A ci-dessus on remplace l'oxyde de titane passivé de l'exemple 1 par de l'oxyde de titane du commerce non traité, la composition ainsi obtenue présente une photostabilité globale caractérisée par :

- un $\Delta E$ égal à 15,
- un taux de pentane d'environ 20.

Il résulte de cette étude comparative que l'utilisation d'un oxyde de titane passivé améliore de façon significative la photostabilité.

Les compositions des exemples suivants présentent également une meilleure stabilité de la coloration dans le temps.

Exemple B

On prépare une émulsion ayant la composition suivante :

- Acide stéarique 2,00 %
- Stéarate de glycéryle 2,90 %
- Isostéarate de glycéryle 2,00 %
- Huile minérale et alcool de lanoline (85/15) 3,50 %
- Palmitate d'isopropyle 5,00 %
- Néopentanoate d'isostéaryle 8,00 %
- Huile de rosier musca 7,50 %
- Cyclométhicone 2,00 %
- Diméthicone 7,00 %
- Propylène glycol 3,00 %
- Silicate de magnésium aluminium 0,80 %

- $TiO_2$ passivé selon l'exemple 1 12,48 %
- Oxydes de fer 2,52 %
- Lauroyl sarcosinate de sodium 3,00 %
- Gomme de cellulose 0,14 %
- Triéthanolamine 1,00 %
- Glycérine 3,00 %
- p-hydroxybenzoate de propyle 0,10 %
- p-hydroxybenzoate de méthyle 0,30 %
- Eau qsp 100,00 %

Exemple C

On prépare une émulsion H/E solaire de haute protection ayant la composition suivante :

- Mélange d'alcool cétylstéarylique et d'alcool cétylstéarylique oxyéthyléné (33 OE) 80/20 7,00 g
- Mélange de mono- et distéarate de glycéryle 2,00 g
- Alcool cétylique 1,50 g
- Polydiméthylsiloxane 1,50 g
- Huile de vaseline 10,00 g
- Benzoate d'alkyle en C12/C15 6,00 g
- p-méthoxycinnamate d'éthyl-2-hexyle 5,00 g
- $TiO_2$ passivé selon l'exemple 1 5,00 g
- ZnO 2,00 g
- Acide téréphtalylidène dicamphre sulfonique 6,00 g
- Triéthanolamine 1,30 g
- Glycérine 20,00 g
- Conservateurs qs
- Parfum qs
- Eau qsp 100,00 g

Exemple D

On prépare un gel/crème solaire ayant la composition suivante :

- Benzoate d'alkyle C12/C15 5,00 g
- tert-butyl-4-méthoxy-4'-dibenzoylméthane 1,00 g
- Cyano-2-diphényl-3,3-acrylate d'éthyl-2-hexyle 5,00 g
- $TiO_2$ passivé selon l'exemple 1 2,00 g
- Acide éthylène diamine tétraacétique, sel disodique, $2H_2O$ 0,10 g
- Terpolymère acide méthacrylique/acrylate d'éthyle/steareth-10 allyl éther (40/50/10) réticulé en émulsion aqueuse à 30 % 5,00 g
- Triéthanolamine 0,75 g
- Glycérine 3,00g
- Conservateurs qs
- Parfum qs
- Eau qsp 100,00 g

**Revendications**

1. Procédé de passivation d'un pigment minéral photoréactif **caractérisé par le fait qu'**il consiste à soumettre ledit pigment minéral photoréactif à un traitement par un plasma froid d'un hydrocarbure perfluoré en $C_1$-$C_4$, à l'exclusion de l'octafluorocyclobutane.

2. Procédé selon la revendication 1, **caractérisé par le fait que** ledit pigment minéral photoréactif est choisi parmi $TiO_2$, ZnO, $Ce_2O_3$, CdS, CaAs, $ZrO_2$, $Fe_2O$, FeO et $Fe_2O_3$.

3. Procédé selon la revendication 1, **caractérisé par le fait que** l'hydrocarbure perfluoré en $C_1$-$C_4$ est le tétrafluo-

rométhane.

4. Procédé selon l'une quelconque des revendications précédentes, **caractérisé par le fait que** la pression de l'hydrocarbure perfluoré en $C_1$-$C_4$, est comprise entre $0,1.10^5$ et 1 Pa.

5. Procédé selon l'une quelconque des revendications précédentes, **caractérisé par le fait que** la durée du traitement est comprise entre 1 et 60 minutes.

6. Utilisation d'un pigment minéral, stable à la lumière, passivé par traitement de passivation par un plasma froid d'un hydrocarbure perfluoré en $C_1$-$C_4$ dans un produit cosmétique ou pharmaceutique.

7. Utilisation selon la revendication 6, **caractérisée par le fait que** le pigment est passivé par le procédé selon l'une des revendications 1 à 5.

8. Composition cosmétique ou pharmaceutique, stable à la lumière, **caractérisée par le fait qu'**elle contient, dans un véhicule approprié, au moins un pigment minéral, sous forme passivée par traitement de passivation par un plasma froid d'un hydrocarbure perfluoré en $C_1$-$C_4$.

9. Composition selon la revendication 8, **caractérisée par le fait que** l'hydrocarbure perfluoré en $C_1$-$C_4$ est le tétrafluorométhane.

10. Composition selon la revendication 8 ou 9, **caractérisée par le fait que** ledit pigment minéral sous forme passivée est présent en une proportion comprise en 0,01 et 40 % en poids par rapport au poids total de la composition.

11. Composition selon l'une quelconque des revendications 8 à 10, **caractérisée par le fait que** ledit pigment minéral sous forme passivée est présent en une proportion comprise entre 0,1 et 25 % en poids par rapport au poids total de la composition.

12. Composition selon l'une quelconque des revendications 8 à 11, **caractérisée par le fait qu'**elle contient en outre au moins un pigment minéral non photoréactif ou un pigment organique en une proportion comprise entre 0,01 et 25 % en poids par rapport au poids total de la composition.

13. Composition selon l'une quelconque des revendications 8 à 12, **caractérisée par le fait qu'**elle présente une photostabilité globale représentée par un photobleuissement $\Delta E = \sqrt{\Delta L^2 + \Delta a^2 + \Delta b^2}$, mesuré à l'aide d'un chromamètre après 1 heure d'irradiation au moyen d'une source lumineuse émettant des rayons UV-A d'intensité 765 $W/m^2$, qui est inférieur à 15.

14. Composition selon l'une quelconque des revendications 8 à 12, **caractérisée par le fait qu'**elle présente une photostabilité globale représentée par un dégagement de pentane en une teneur inférieure à 20 µg, après photooxydation, en présence d'un réactif comportant des chaînes linoléiques, au moyen d'une source lumineuse émettant des rayons UV-A d'intensité 89 $W/m^2$ et des rayons UV-B d'intensité 0,05 $W/m^2$, pendant au plus 4 heures.

15. Composition selon l'une quelconque des revendications 8 à 14, **caractérisée par le fait qu'**elle se présente sous une forme choisie parmi les fard à paupières, eye-liner, mascara, poudre de maquillage, fond de teint, fard à joues, blush, crème teintée, rouge à lèvres, stick à lèvres, stick anti-cernes, produit solaire, crème, vernis à ongles anhydres et vernis à ongles aqueux.

## Patentansprüche

1. Verfahren zur Passivierung eines fotoreaktiven Mineralpigments, **dadurch gekennzeichnet, dass** es aus dem Unterwerfen des fotoreaktiven Mineralpigments unter eine Behandlung mit einem kalten Plasma eines perfluorierten $C_1$-$C_4$-Kohlenwasserstoffs, ausgenommen Octafluorcyclobutan, besteht.

2. Verfahren gemäß Anspruch 1, **dadurch gekennzeichnet, dass** das fotoreaktive Mineralpigment ausgewählt ist unter $TiO_2$, ZnO, $Ce_2O_3$, CdS, CaAs, $ZrO_2$, $Fe_2O$, FeO und $Fe_2O_3$.

3. Verfahren gemäß Anspruch 1, **dadurch gekennzeichnet, dass** der perfluorierte $C_1$-$C_4$-Kohlenwasserstoff das

Tetrafluormethan ist.

4. Verfahren gemäß einem der vorstehenden Ansprüche, **dadurch gekennzeichnet, dass** der Druck der perfluorierten $C_1$-$C_4$-Kohlenwasserstoffs zwischen 0,1 x $10^5$ und 1 Pa liegt.

5. Verfahren gemäß einem der vorstehenden Ansprüche, **dadurch gekennzeichnet, dass** die Behandlungsdauer zwischen 1 und 60 Minuten liegt.

6. Verwendung eines im Licht stabilen Mineralpigments, passiviert über die Passivierungsbehandlung durch ein kaltes Plasma eines perfluorierten $C_1$-$C_4$-Kohlenwasserstoffs, in einem kosmetischen oder pharmazeutischen Produkt.

7. Verwendung gemäß Anspruch 6, **dadurch gekennzeichnet, dass** das Pigment über das Verfahren nach einem der Ansprüche 1 bis 5 passiviert ist.

8. Kosmetische oder pharmazeutische Zubereitung, die im Licht stabil ist, **dadurch gekennzeichnet, dass** sie in einem geeigneten Träger mindestens ein Mineralpigment in durch Behandlung zur Passivierung mit Hilfe eines kalten Plasmas einer perfluorierten $C_1$-$C_4$-Kohlenwasserstoffs passivierter Form enthält.

9. Zubereitung gemäß Anspruch 8, **dadurch gekennzeichnet, dass** der perfluorierte $C_1$-$C_4$-Kohlenwasserstoff das Tetrafluormethan ist.

10. Zubereitung gemäß Anspruch 8 oder 9, **dadurch gekennzeichnet, dass** das Mineralpigment in passivierter Form in einer Menge zwischen 0,01 und 40 Gew.-% vorliegt, bezogen auf das Gesamtgewicht der Zubereitung.

11. Zubereitung gemäß einem der Ansprüche 8 bis 10, **dadurch gekennzeichnet, dass** das Mineralpigment in passivierter Form in einer Menge zwischen 0,1 und 25 Gew.-% vorliegt, bezogen auf das Gesamtgewicht der Zubereitung.

12. Zubereitung gemäß einem der Ansprüche 8 bis 11, **dadurch gekennzeichnet, dass** diese zusätzlich mindestens ein nicht fotoreaktives Mineralpigment oder ein organisches Pigment in einer Menge zwischen 0,01 und 25 Gew.-% enthält, bezogen auf das Gesamtgewicht der Zubereitung.

13. Zubereitung gemäß einem der Ansprüche 8 bis 12, **dadurch gekennzeichnet, dass** sie eine Gesamtfotostabilität aufweist, dargestellt über eine Fotobläuung von $\Delta E = \sqrt{\Delta L^2 + \Delta a^2 + \Delta b^2}$, gemessen mit Hilfe eines Chromometers nach einer Stunde Bestrahlung mit Hilfe einer Lichtquelle, die UV-A-Strahlen einer Intensität von 765 W/$m^2$ emittiert, die unterhalb von 15 liegt.

14. Zubereitung gemäß einem der Ansprüche 8 bis 12, **dadurch gekennzeichnet, dass** sie eine Gesamtfotostabilität aufweist, repräsentiert durch eine Pentanfreisetzung in einem Gehalt unterhalb von 20 µg, nach Fotooxidation in Anwesenheit eines Linoleinketten tragenden Reagenzes mit Hilfe einer Lichtquelle, die UV-A-Strahlen einer Intensität von 89 W/$m^2$ und UV-B-Strahlen einer Intensität von 0,05 W/$m^2$ emittiert, für mehr als 4 Stunden.

15. Zubereitung gemäß einem der Ansprüche 8 bis 14, **dadurch gekennzeichnet, dass** sie in einer Form vorliegt, ausgewählt unter Lidschatten, Eyeliner, Mascara, Schminkpuder, Grundierung, Rouge, Blush, getönter Creme, Lippenrot, Lippenstiften, Stiften gegen Augenringe, Sonnenprodukten, Creme, wasserfreiem Nagellack und wässrigem Nagellack.

## Claims

1. Process for passivating a photoreactive mineral pigment, **characterized in that** it consists in subjecting the said photoreactive mineral pigment to a treatment with a cold plasma of a $C_1$-$C_4$ perfluorohydrocarbon, with the exclusion of octafluorocyclobutane.

2. Process according to Claim 1, **characterized in that** the said photoreactive mineral pigment is chosen from $TiO_2$, ZnO, $Ce_2O_3$, CdS, CaAs, $ZrO_2$, $Fe_2O$, FeO and $Fe_2O_3$.

3. Process according to Claim 1, **characterized in that** the $C_1$-$C_4$ perfluorohydrocarbon is tetrafluoromethane.

4. Process according to any one of the preceding claims, **characterized in that** the pressure of the $C_1$-$C_4$ perfluorohydrocarbon is between $0.1 \times 10^5$ and 1 Pa.

5. Process according to any one of the preceding claims, **characterized in that** the duration of the treatment is between 1 and 60 minutes.

6. Use of a light-stable mineral pigment, passivated by means of a passivation treatment with a cold plasma of a $C_1$-$C_4$ perfluorohydrocarbon in a cosmetic or pharmaceutical product.

7. Use according to Claim 6, **characterized in that** the pigment is passivated by the process according to one of Claims 1 to 5.

8. Light-stable cosmetic or pharmaceutical composition, **characterized in that** it contains, in a suitable vehicle, at least one mineral pigment, in a form passivated by means of a passivation treatment with a cold plasma of a $C_1$-$C_4$ perfluorohydrocarbon.

9. Composition according to Claim 8, **characterized in that** the $C_1$-$C_4$ perfluorohydrocarbon is tetrafluoromethane.

10. Composition according to Claim 8 or 9, **characterized in that** the said mineral pigment in passivated form is present in a proportion of between 0.01% and 40% by weight relative to the total weight of the composition.

11. Composition according to any one of Claims 8 to 10, **characterized in that** the said mineral pigment in passivated form is present in a proportion of between 0.1% and 25% by weight relative to the total weight of the composition.

12. Composition according to any one of Claims 8 to 11, **characterized in that** it also contains at least one non-photoreactive mineral pigment or an organic pigment in a proportion of between 0.01% and 25% by weight relative to the total weight of the composition.

13. Composition according to any one of Claims 8 to 12, **characterized in that** it has an overall photo-stability, represented by a photoblueing $\Delta E=\sqrt{\Delta L^2 + \Delta a^2 + \Delta b^2}$ measured using a chromameter after one hour of irradiation using a light source that emits UV-A rays with an intensity of 765 W/$m^2$, which is less than 15.

14. Composition according to any one of Claims 8 to 12, **characterized in that** it has an overall photostability, represented by a release of pentane in an amount of less than 20 µg, after photo-oxidation, in the presence of a reagent comprising linoleic chains, using a light source that emits UV-A rays with an intensity of 89 W/$m^2$ and UV-B rays with an intensity of 0.05 W/$m^2$, for not more than four hours.

15. Composition according to any one of Claims 8 to 14, **characterized in that** it is in a form chosen from eyeshadows, eyeliners, mascaras, makeup powders, foundations, face powders, blushers, tinted creams, lip colourings, lipsticks, concealer sticks, antisun products, creams, anhydrous nail varnishes and water-based nail varnishes.